# EUROPEAN PATENT APPLICATION

(11) **EP 0 990 633 A1**
(43) Date of publication of application: **05.04.2000**
(21) Application number: 98911225.5
(22) Date of filing: 07.04.1998
(51) Int. Cl.: C07C 39/17

(54) **PROCESSES FOR PRODUCING p-SUBSTITUTED PHENOLS**

(30) Priority: 08.04.1997 JP 10529397
(71) Applicant: CHISSO CORPORATION, Osaka-shi, Osaka-fu 530-0005 (JP)
(72) Inventor: HASEBA, Yasuhiro, Matsudo-shi Chiba 270-2211 (JP); MIYAZAWA, Kazutoshi, Chiba 276-0022 (JP); TAKEUCHI, Hiroyuki, Chiba 290-0022 (JP); TATEIWA, Junichi, Sakyo-ku Kyoto-shi Kyoto 606-8416 (JP); UEMURA, Sakae, Kyoto-shi Kyoto 606-0841 (JP)
(74) Representative: Ziebig, Marlene, Dr. Dipl.-Chem.
(86) International application number: JP9801590
(87) International publication number: WO9845234

(57) **Abstract**

Para-substituted phenols are produced at high yield and high selectivity without generating a large quantity of a metal containing drain, and further, para-substituted cyclohexylphenols in which the substituents at position 1 and position 4 of cyclohexanone are in trans relation are produced at high selectivity when a cyclohexanone having a substituent at position 4 is used as reaction substrate.

A process for producing a para-substituted phenol comprising the step of reacting a phenol with a compound having carbonyl group by using a clay mineral as catalyst.

## Description

### TECHNICAL FIELD

The present invention relates to a process for producing a para-substituted phenol which can be employed mainly as material for synthesizing liquid crystal compounds, and others. More specifically, the present invention relates to a process for producing a para-substituted alkylphenol (hereinafter, the term "alkyl" in the word "alkylphenol" includes a cyclohexyl having a substituent at least at position 4 unless specified as straight chain alkyl or branched alkyl, and in this case, the substituent at position 4 of cyclohexane ring is not restricted to an alkyl group) from a compound having carbonyl group and a phenol by using a clay mineral as catalyst.

### BACKGROUND ART

Heretofore, as a process for producing an alkylphenol, a method is well known in which an alcohol formed by the Grignard reaction of a halogen substituted phenol in which hydroxyl group is protected, with a ketone or aldehyde is dehydrated with an acid, the double bond formed by the dehydration is hydrogenated, and then deprotected. This method, however, has the defects of (a) to (c) shown below:
(a) Process is long,
(b) A large quantity of a drain containing a metal is generated, and
(c) Cyclohexane ring becomes a mixture of cis-form and trans-form after hydrogenation when 4-alkylcyclohexanone is used.

Technology for solving the (a) and (b) among these defects is described in Laid-open Japanese Patent Publication No. Hei 7-41445. This technology is to obtain 4-(4-n-alkylcyclohexyl)-phenol in one step by subjecting a phenol and a straight chain alkylcyclohexanone to coupling reaction by using a heteropoly-acid as catalyst. However, Laid-open Japanese Patent Publication No. Hei 7-41445 does not include any suggestion for solving the defect (c) described above, and besides, the amount of the compound which is formed by the side reaction and has an alkylcyclohexyl group introduced at ortho-position to the hydroxyl group of the phenol is unknown from the publication.

Accordingly, literatures have not been published which suggest a process for producing a cyclohexylphenol derivative in which the relation between the substituents at position 1 and position 4 of cyclohexane is trans, in one step at high selectivity and high yield without generation of a large quantity of a metal containing drain.

Method by which a straight chain alkylphenol or cyclohexylphenol is obtained from a phenol, and a straight chain aldehyde or cyclohexanone in one step, respectively, is described in Chemistry Letters 1996, page 59. In this method, whereas a straight chain aldehyde or cyclohexanone is reacted with a phenol by using a montmorillonite exchanged with Al³⁺ as catalyst, the main product is a bisphenol derivative when a straight chain aldehyde is used, and about 20 % of an ortho-substituted cyclohexylphenol is produced as by-product when cyclohexanone is used. Accordingly, a process by which a para-substituted alkylphenol is obtained from an aldehyde in one step at high selectivity and high yield without generation of a large quantity of a metal containing drain has not been found, and an example in which cyclohexyl group is selectively introduced at para-position of a phenol by using a cyclohexanone derivative has not been proposed. Further, literatures have not been published through which the selectivity of the cis-form compound and trans-form compound in the reaction using a compound having a substituent at position 4 of cyclohexanone can be predicted.

### DISCLOSURE OF THE INVENTION

Subject of the present invention is to produce a para-substituted phenol at high yield and high selectivity without generation of a large quantity of a metal containing drain, and further, to produce a para-substituted cyclohexylphenol in which the substituents at position 1 and 4 is in trans relation, at high selectivity when cyclohexanone having a substituent at position 4 is used as reaction substrate.

As a result of diligent investigations to solve the subjects described above by the present inventors, a method has been found by which a cyclohexylphenol which has cyclohexane ring at para-position to the hydroxyl group of a phenol and in which the substituents at position 1 and position 4 of cyclohexane are in trans relation is produced at high selectivity and high yield from a cyclohexanone derivative having a substituent at position 4 by using a clay mineral or montmorillonite as catalyst.

The present invention is summarized as follows:
(1) A process for producing a para-substituted phenol comprising the step of reacting a phenol with a compound having carbonyl group using a clay mineral as catalyst.
(2) The process for producing a para-substituted phenol recited in paragraph (1) above wherein the compound having carbonyl group is a cyclohexanone derivative expressed by the general formula (1) or an aldehyde expressed by the general formula (2), and the phenol is a phenol expressed by the general formula (3) 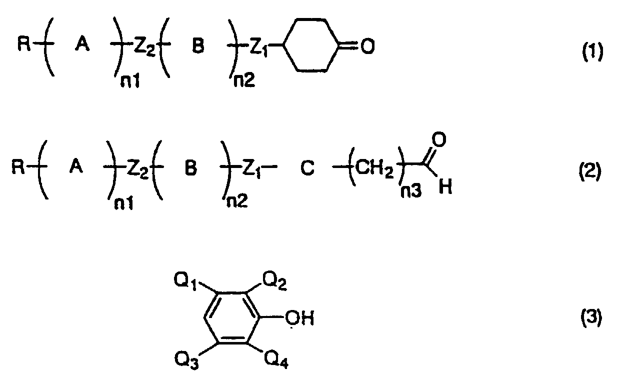 in the general formula (1) or (2), A, B, and C each independently represent a carbon six-membered ring in which ring one or more carbon atoms may be replaced by hetero atoms, and one or more hydrogen atoms may be replaced by halogen atoms, alkyl groups, cyano groups, or hydroxyl groups; R represents hydroxyl group, a halogen atom, cyano group, amino group, nitro group, -CH=CF₂, -CH₂CH₂CH=CF₂, or a straight chain or branched alkyl group having 1 to 30 carbon atoms in which alkyl group methylene group may be replaced by oxygen atom, sulfur atom, -COO-, -OCO-, -CH=CH-, or -C≡C-, but there is not a case that oxygen atoms are adjacent, and one or more hydrogen atoms in which group may be replaced by halogen atoms, hydroxyl groups, cyano groups, or nitro groups; Z₁ and Z₂ each independently represent single bond, -CH₂-, -CH₂CH₂-, -CH₂CH₂CH₂-, -CH₂CH₂CH₂CH₂-, -CH(CH₃)CH₂-, -CH₂CH(CH₃)-, -CH₂O-, -OCH₂-, -CH₂CH₂CH₂O-, -OCH₂CH₂CH₂-, -COO-, -OCO-, -CF₂O-, -OCF₂-, -CH=CH-, -CF=CF-, -C≡C-, or -S-S-; n1 and n2 are each independently 0 or 1; and n3 is an integer of 0 to 5;
   in the general formula (3), Q₁ to Q₄ each independently represent hydrogen atom, a halogen atom, an alkyl group, or cyano group;
   and
   each atom which constitutes these compounds may be replaced by its isotope.
(3) The process for producing a para-substituted phenol recited in paragraph (1) or (2) above wherein the clay mineral is an ion exchange type clay mineral.
(4) The process for producing a para-substituted phenol recited in paragraph (1) or (2) above wherein the clay mineral is a metal exchange type clay mineral.
(5) The process for producing a para-substituted phenol recited in paragraph (1) or (2) above wherein the clay mineral is an ion exchange type montmorillonite.
(6) The process for producing a para-substituted phenol recited in paragraph (1) or (2) above wherein the clay mineral is a metal exchange type montmorillonite.
(7) The process for producing a para-substituted phenol recited in paragraph (3) above wherein the ion exchange type clay mineral is an ion exchange type montmorillonite expressed by the general formula (4)

   (Xa)m1(Xb)m2(Xc₄O₁₀)(Xd)₂ · mH₂O (4)

   in the general formula (4), Xa represents at least one ion selected from the group consisting of a cation of the elements of group 1, group 2, group 3, group 4, group 5, group 6, group 7, group 8, group 9, group 10, group 11, group 12, group 13, group 14, or group 15, and ammonium ion; Xb represents at least one atom selected from the group consisting of lithium, magnesium, aluminum, cadmium, lead, zinc, copper, iron, cobalt, nickel, and manganese; Xc represents a silicon atom, a part of which may be replaced by at least one atom selected from the group consisting of aluminum, iron, germanium, titanium, and boron; Xd represents fluorine atom or OH; m1 and m2 are numerical values determined by electrostatic balance, and m1 is a number of 1/5 to 1 and m2 is a number of 1 to 3; and m indicates the amount of water of hydration.
(8) The process for producing a para-substituted phenol recited in paragraph (7) above wherein Xa in the general formula (4) is at least one cation selected from the group consisting of H⁺, Na⁺, Sn⁺, Ca²⁺, Mg²⁺, Zn²⁺, Ce²⁺, Cu²⁺, Pb²⁺, Ni²⁺, Al³⁺, Fe³⁺, and Zr⁴⁺.
(9) A process for producing a para-substituted phenol comprising the step of reacting a cyclohexanone derivative expressed by the general formula (1) with a phenol expressed by the general formula (3) by using an ion exchange type montmorillonite expressed by the general formula (4) as catalyst.
(10) The process for producing a para-substituted phenol recited in paragraph (9) above wherein Xa in the general formula (4) is at least one cation selected from the group consisting of H⁺, Na⁺, Sn⁺, Ca²⁺, Mg²⁺, Zn²⁺, Ce²⁺, Cu²⁺, Pb²⁺, Ni²⁺, Al³⁺, Fe³⁺, and Zr⁴⁺.
(11) A process for producing a para-substituted phenol comprising the step of reacting an aldehyde expressed by the general formula (2) with a phenol expressed by the general formula (3) by using an ion exchange type montmorillonite expressed by the general formula (4) as catalyst. (12) The process for producing a para-substituted phenol recited in paragraph (11) above wherein Xa in the general formula (4) is at least one cation selected from the group consisting of H⁺, Na⁺, Sn⁺, Ca²⁺, Mg²⁺, Zn²⁺, Ce²⁺, Cu²⁺, Pb²⁺, Ni²⁺, Al³⁺, Fe³⁺, and Zr⁴⁺.

The present invention is described in more detail below. In the general formula (1) or (2), A, B, and C are as described above, and specifically following ring structures can be mentioned as their examples, but they are not restricted to those examples.

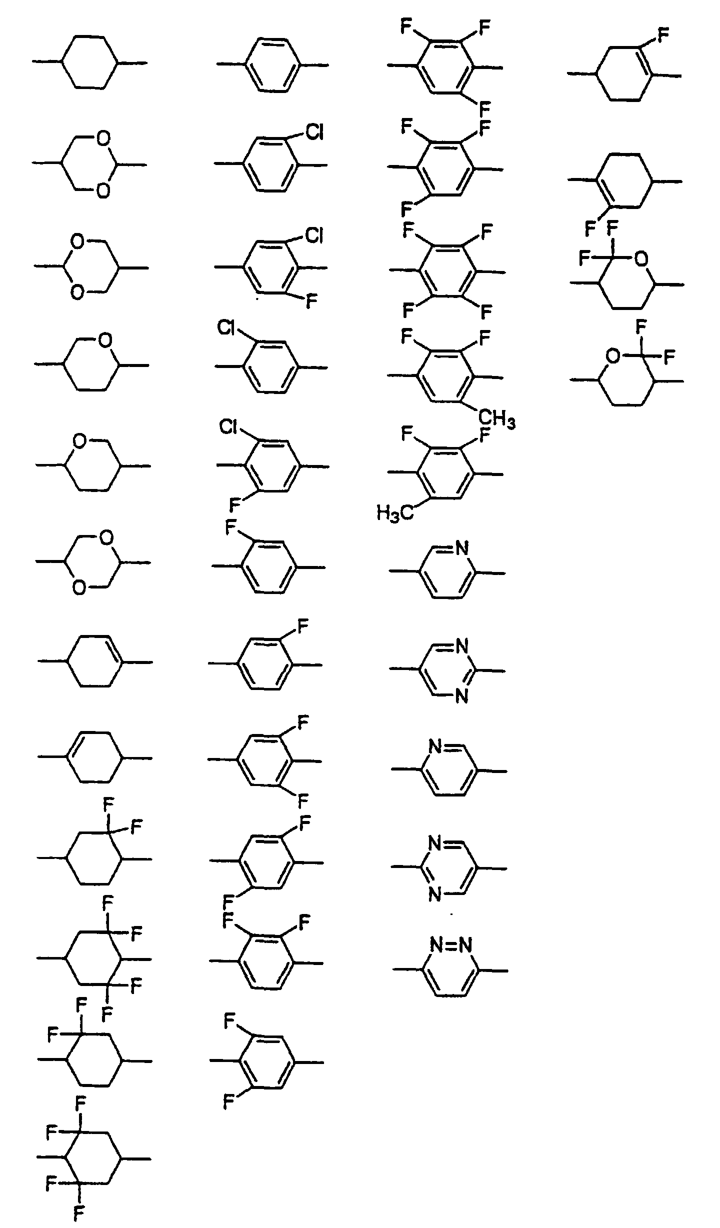

In the general formulas (1) and (2), R is as described above, and specifically methyl group, ethyl group, n-propyl group, n-butyl group, n-pentyl group, n-hexyl group, n-heptyl group, n-octyl group, n-nonyl group, n-decanyl group, methoxy group, ethoxy group, n-propoxy group, n-butoxy group, n-pentoxy group, n-hexaoxy group, n-heptaoxy group, methoxymethyl group, ethoxymethyl group, n-propoxymethyl group, n-butoxymethyl group, n-pentoxymethyl group,methoxyethyl group, ethoxyethyl group, n-propoxyethyl group, n-butoxyethyl group, n-pentoxyethyl group, fluoromethyl group, difluoromethyl group, trifluoromethyl group, 1-fluoroethyl group, 2-fluoroethyl group, 1,1-difluoroethyl group, 1,2-difluoroethyl group, 2,2-difluoroethyl group, 1,1,2-trifluoroethyl group, 1,2,2-trifluoroethyl group, 2,2,2-trifluoroethyl group, 3-fluoropropyl group, 2-fluoropropyl group, 1-fluoropropyl group, 4-fluorobutyl group, 5-fluoropentyl group, trifluoromethoxy group, difluoromethoxy group, fluoromethoxy group, 1,1,2,2-tetrafluoroethoxy group, 1,1,2,3,3,3-hexafluoropropoxy group, 2,2-difluorovinyl group, 1,2,2-trifluorovinyl group, vinyl group, 3-butenyl group, 1-butenyl group, 2-methylbutyl group, and 2-trifluoromethylbutyl group can be mentioned as its examples, but it is not restricted to these groups. Besides, when the compound expressed by the general formula (1) or (2) is a branched alkyl compound having an asymmetric carbon atom, it may be an optically active compound or racemic modification.

As the phenol expressed by the general formula (3), the following can be mentioned as examples, but it is not restricted to those examples.

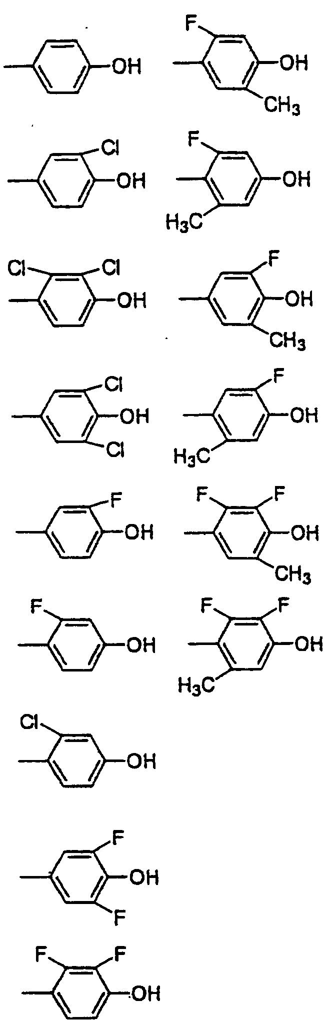

Now, the clay mineral used in the present invention is described. Characteristic mineral which constitutes a clay is a phyllosilicate mineral, and mainly kaolin, montmorillonite, clay mica, and chlorite belong thereto. In clay minerals, many times these various kind of phyllosilicates form a mixed layer, and they are called mixed layer minerals. In this specification, the term "clay mineral" refers to a mixture of unspecified ratio of various kind of phyllosilicates described above, it does not exclude the cases where montmorillonite is contained.

Phyllosilicate minerals form a layer structure comprising a tetrahedral sheet formed by a method in which three oxygens in a SiO₄ tetrahedron are shared with the adjacent tetrahedron and link in a state of hexagonal reticulation, and an octahedral sheet formed by oxygen and OH ion coordinated to a metal ion such as Al, Mg, and Fe. Remaining one oxygen atom in the tetrahedral sheet stands in a line facing the same direction and is shared with the adjacent octahedral sheet.

Montmorillonite is one of typical clay minerals, and the crystal structure of montmorillonite has a layer formed by inserting an alumina octahedral sheet between two tetrahedral sheets, as basic unit layer. In more detail, in a tetrahedral sheet, a tetrahedron in which four O²⁻ surround Si⁴⁺ shares three apexes with the adjacent tetrahedron to link in a state of a hexagonal reticulation. Remaining one apex stands in a line facing the same direction and is held in common together with apexes of the adjacent octahedron sheet. On the other hand, the octahedral sheet is one in which octahedrons having a central cation and six OH⁻ or O²⁻ surrounding the cation share their edge and two-dimensionally spread, an apex occupied by O²⁻ shares apex oxygen in upper and lower tetrahedrons, and there is no linking with the adjacent sheet at the position of OH⁻. Central cation, Al³⁺ exists at 2/3 of cation sites in the octahedron, and remaining 1/3 are vacant sheets.

Unit layer of this montmorillonite is in a state of a flake having a thickness of about 10 A and a stretch of 0.1 to 1 µm, and several sheets of the unit layers pile up to form a mineral particle. Accordingly, montmorillonite particles can be said to be particles in a state of a thin layer having the ratio of thickness to diameter of 1:100 to 1:200.

In montmorillonite, a part of Al³⁺ ion which is a central trivalent cation in a octahedral sheet is replaced by a divalent ion having nearly the same ion radius such as Mg²⁺ ion. By this replacement, the unit layer structure comes to have a negative electrical charge and comes to have a quality of a solid acid. This negative electrical charge is neutralized with Na⁺, Ca²⁺, or H₃O⁺ absorbed between the layers. These ions are called interlayer cations. When montmorillonite is immersed in a solution containing another ion, an ion exchange reaction with the interlayer cation is quickly occasioned, and the interlayer distance of montmorillonite is varied due to difference in the dimensions of the exchanged cation.

Clay mineral in which an interlayer cation is exchanged with a desired ion is called ion exchange type clay mineral, the montmorillonite in which an interlayer cation is exchanged with a desired ion is called ion exchange type montmorillonite, and the introduced cation is called exchanging cation. On the other hand, a clay mineral or montmorillonite in which a part of silicon, aluminum, or magnesium which is a central atom in octahedron or tetrahedron is exchanged with another metal is called metal exchange type clay mineral or metal exchange type montmorillonite, respectively.

As described above, a clay mineral is characterized in that its acidic property can readily be changed by the ion exchange and that various molecules can be taken as guest molecule in an extremely small space in the order of nanometer formed by the same group of clay mineral layers. Also in montmorillonite, its property as solid acid and interlayer distance are changed depending on the type of exchanging cation. Accordingly, it has a characteristic that the property of montmorillonite as solid acid catalyst can be optimized by selecting an exchanging cation according to the objective reaction.

Clay mineral and montmorillonite used as catalyst in the present invention may be a natural product or a synthetic product. Synthetic clay mineral and synthetic montmorillonite can be obtained by conventional methods.

Ion exchange type clay mineral and ion exchange type montmorillonite used in the present invention may be a product of single component or a mixture of two or more kind of products. Among them, an ion exchange type montmorillonite of a single component is preferable since the selectivity of reaction is good and yield of a para-substituted alkylphenol is excellent when a single component ion exchange type montmorillonite is used.

Ion exchange type montmorillonite can be produced by a method described in Clays Clay Miner, vol. 27, page 119 (published in 1979) and a modified method thereof. For instance, it can be obtained by immersing a commercially available montmorillonite in which the interlayer cation is Na⁺ (Na⁺-mont.) (hereinafter, montmorillonite ion exchanged with Mⁿ⁺ is referred to as Mⁿ⁺-mont.) in a solution of a salt, for example, sulfate, nitrate, or organic acid salts of a metal which is preferred for substituting. Acid site of the clay mineral and montmorillonite thus obtained can be determined by ammonia gas analysis.

The ion exchange type montmorillonite used as catalyst in the present invention is expressed by the following general formula (4)

(Xa)m1(Xb)m2(Xc₄O₁₀)(Xd)₂ · mH₂O (4)

in the general formula (4), Xa represents an ion exchanging cation which was used in ion exchange for the interlayer cation, specifically it is a cation of elements of group 1 to elements of group 15, or cation of ammonium, and it may sometimes be in a hydrated condition; Xb represents aluminum atom, which is a central atom of octahedron, a part of which may be replaced by at least one atom selected from the group consisting of lithium, magnesium, cadmium, lead, zinc, copper, iron, cobalt, nickel, and manganese; Xc represents a silicon atom, which is a central atom of tetrahedron, a part of which may be replaced by at least one atom selected from the group consisting of aluminum, iron, germanium, titanium, and boron; Xd represents fluorine atom or OH; m1 and m2 are numerical values determined by electrostatic balance, and m1 is a number of 1/5 to 1 and m2 is a number of 1 to 3; and m indicates the amount of water of hydration.

In the general formula (4), the interlayer cation represented by Xa is preferably H⁺, Na⁺, Sn⁺, Ca²⁺, Mg²⁺, Zn²⁺, Ce²⁺, Cu²⁺, Pb²⁺, Ni²⁺, Al³⁺, Fe³⁺, and Zr⁴⁺, and more desirably Sn⁺, Zn²⁺, Ce²⁺, Cu²⁺, Pb²⁺, Ni²⁺, Al³⁺, Fe³⁺, and Zr⁴⁺.

There is no specific restriction in the reaction conditions between a phenol and a compound having carbonyl group. While there is no specific restriction even in the molar ratio of reactants, it is preferable to use an excess amount of a phenol since reaction time can be shortened. It is more desirable to use a phenol which is 1.2 times to 35 times as large amount as a compound having carbonyl group. Further, while there is no specific restriction in the molar number of acid site of a clay mineral or montmorillonite, it is preferably in the range of 1/1000 to 1/1 and more desirably in the range of 1/100 to 1/1 based on the molar number of a compound having carbonyl group. As reaction solvent, one which is not involved in the reaction is used. For example, benzene, toluene, xylene, chlorobenzene, dimethoxyethane, and dioxane can be used. However, since the reaction sufficiently proceeds even in the absence of a solvent, the solvent is not necessarily required to use.

While the reaction temperature can be selected without restraint so far as reactants, products, and catalysts are not remarkably deteriorated, it is usually in the range of room temperature to 280°C and more preferably 50°C to 150°C. Reaction sufficiently proceeds even under normal pressure, but reaction may be conducted under an increased pressure. While the reaction time largely depends on the type of a catalyst, phenol, aldehyde, and ketone as well as their amount to be used, reaction temperature, and pressure, usually it is 0.1 to 200 hours.

In order to isolate a product (alkylphenol) from a reaction system after termination of the reaction, methods which can be employed in ordinary chemical operations are used. For instance, a catalyst is filtered off from a reaction liquid, the filtrate is subjected to distillation and chromatography to obtain an objective product, and can be purified by conducting an operation such as recrystallization, if necessary. The catalyst separated by filtration can be reused after washing and drying.

Virtually all of the product (alkylphenol) obtained by the reaction of the present invention are para-substituted alkylphenol. Specifically, ortho-substituted product is less than 10 %, and in nearly all cases it is a very small quantity or 0 %. Further, when carbonyl compound is a cyclohexanone derivative, almost all of the substituents at position 1 and position 4 of cyclohexane ring are in trans relation. Specifically, products in cis-relation are less than 3 %, and in nearly all cases it is a very small quantity or 0 %. Since the reaction proceeds selectively as described above, isolation of an objective product is easy and besides the yield is high.

### BEST MODE FOR CARRYING OUT THE INVENTION

Now, the present invention will be described in more detail with reference to Examples. However, it should be understood that the scope of the present invention is by no means restricted by such specific Examples.

### Example 1 (Preparation Example 1 of catalyst)

In an Erlenmeyer flask having a content volume of 2 L, 12 g of montmorillonite which is ion exchanged with sodium (Na⁺-mont.) (Trade name: Kunipia G, produced by Kunimine Industrial Co., Ltd.) was suspended in 400 ml of water and 400 ml of acetone, and stirred for 24 hours. This suspension was heated at 50°C, and an aqueous solution prepared by dissolving 45 g of aluminum nitrate nonahydrate in 1.2 L of water was gradually added to the suspension and stirred at the same temperature for 24 hours. After it was allowed to stand at room temperature for 30 minutes to cool, the suspension was subjected to filtration. The cake thus obtained was washed with 1 L of water once and 400 ml of water seven times, dried at 80°C under normal pressure for 24 hours, and then pulverized into fine particles. The particles were sifted through a sieve of 100 mesh and then dried at 120°C under normal pressure for 24 hours to obtain 10 g of montmorillonite ion exchanged with aluminum (Al³⁺-mont.).

### Example 2 (Preparation Example 2 of catalyst)

Example 1 was repeated with the exception that 39 g of zirconium chloride octahydrate was used in place of the aluminum nitrate nonahydrate used in Example 1 to obtain 10 g of montmorillonite ion exchanged with zirconium (Zr⁴⁺-mont.).

### Example 3

Drying tube was equipped to a two neck flask having a content volume of 20 ml, 0.50 g of the Al³⁺-mont. (molar number of acid site determined by ammonia gas analysis was 0.266 mmol) prepared in Example 1 and 2.5 g (26.6 mmol) of phenol were added to the flask, heated at 100°C, 0.11 g (0.78 mmol) of 4-n-propylcyclohexanone was added, and then stirred at the same temperature for 48 hours. After the reaction product was cooled down to room temperature, it was dissolved by adding 5 ml of ether thereto, and then subjected to filtration. After the solid on the filter paper was washed with ether, the ether was distilled off under a reduced pressure. The residue was subjected to Kugelrohr distillation under a reduced pressure to remove, first, phenol at 150°C under a pressure of 3 torr. After the receiver was exchanged, the distillate at a kettle temperature of 250°C under a pressure of 3 torr was collected to obtain 0.061 g (0.28 mmol) of a mixture of 4-(4-n-propylcyclohexyl)phenol and 2-(4-n-propylcyclohexyl)phenol in which cyclohexane ring the substituents at position 1 and position 4 are in trans relation. Yield of this product was 36 %. According to G. C. mass spectrum analysis, the ration of 4-(4-n-propylcyclohexyl)-phenol to 2-(4-n-propylcyclohexyl)phenol was found 9 to 1.

### Example 4

Example 3 was repeated with the exception that Zr⁴⁺-mont. was used in place of Al³⁺-mont. used in Example 3 to obtain 0.034 g (0.15 mmol) of 4-(4'-n-propylcyclohexyl)phenol in which cyclohexane ring the substituents at position 1 and position 4 are in trans relation. Yield of this product was 20 %. Ortho-substituted compound was not formed.

### Example 5

Example 3 was repeated with the exception that 0.087 g (0.78 mmol) of 4-methylcyclohexanone was used in place of 4-n-propylcycloheanone used in Example 3 to obtain 0.067 g (0.35 mmol) of 4-(4-methylcyclohexyl)phenol in which cyclohexane ring the substituents at position 1 and position 4 are in trans relation. Yield of this compound was 45 %.

### Example 6

Example 3 was repeated with the exception that 0.12 g (0.78 mmol) of 4-t-butylcyclohexanone was used in place of 4-n-propylcycloheanone used in Example 3 to obtain 0.076 g (0.33 mmol) of 4-(4-t-butylcyclohexyl)phenol in which cyclohexane ring the substituents at position 1 and position 4 are in trans relation. Yield of this compound was 42 %.

### Example 7

Example 3 was repeated with the exception that 0.20 g of 2-(4'-(4''-n-propylcyclohexyl)cyclohexyl)ethanal was used in place of 4-n-propylcycloheanone used in Example 3 to obtain 4-(2-(4'-(4''-n-propylcyclohexyl)cyclohexyl)ethyl)phenol in which cyclohexane ring the substituents at position 1 and position 4 are in trans relation. In this reaction, it was confirmed by gas chromatography that bisphenols were not formed.

### INDUSTRIAL APPLICABILITY

By using the production process of the present invention, it is possible to produce para-substituted phenols in one step at high yield and high selectivity without generation of a large quantity of a metal containing drain, and further, it is possible to produce para-substituted cyclohexylphenols in which cyclohexane ring the substituents at position 1 and position 4 are in trans relation, at high selectivity, when a cyclohexanone having a substituent at position 4 is used as reaction substrate.

## Claims

1. A process for producing a para-substituted phenol comprising the step of reacting a phenol with a compound having carbonyl group by using a clay mineral as catalyst.

2. The process for producing a para-substituted phenol according to claim 1 wherein the compound having carbonyl group is a cyclohexanone derivative expressed by the general formula
(1) or an aldehyde expressed by the general formula (2), and the phenol is one expressed by the general formula (3) 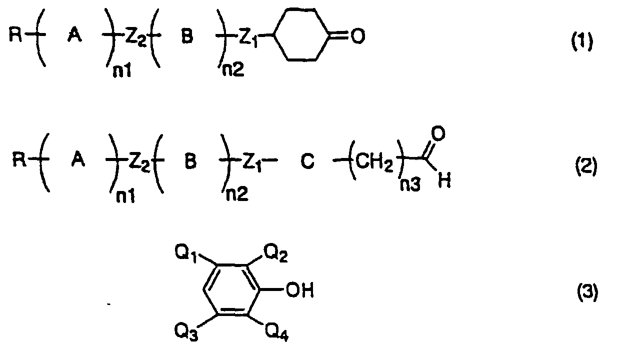 in the general formula (1) or (2), A, B, and C each independently represent a carbon six-membered ring in which ring one or more carbon atoms may be replaced by hetero atoms, and one or more hydrogen atoms may be replaced by halogen atoms, alkyl groups, cyano groups, or hydroxyl groups; R represents hydroxyl group, a halogen atom, cyano group, amino group, nitro group, -CH=CF₂, -CH₂CH₂CH=CF₂, or a straight chain or branched alkyl group having 1 to 30 carbon atoms in which alkyl group methylene group may be replaced by oxygen atom, sulfur atom, -COO-, -OCO-, -CH=CH-, or -C≡C-, but there is not a case that oxygen atoms are adjacent, and one or more hydrogen atoms in which group may be replaced by halogen atoms, hydroxyl groups, cyano groups, or nitro groups; Z₁ and z₂ each independently represent single bond, -CH₂-, -CH₂CH₂-, -CH₂CH₂CH₂-, -CH₂CH₂CH₂CH₂-, -CH(CH₃)CH₂-, -CH₂CH(CH₃)-, -CH₂O-, -OCH₂-, -CH₂CH₂CH₂O-, -OCH₂CH₂CH₂-, -COO-, -OCO-, -CF₂O-, -OCF₂-, -CH=CH-, -CF=CF-, -C≡C-, or -S-S-; n1 and n2 are each independently 0 or 1; and n3 is an integer of 0 to 5;
in the general formula (3), Q₁ to Q₄ each independently represent hydrogen atom, a halogen atom, an alkyl group, or cyano group;
and
each atom which constitutes these compounds may be replaced by its isotope.

3. The process for producing a para-substituted phenol according to claim 1 or 2 wherein the clay mineral is an ion exchange type clay mineral.

4. The process for producing a para-substituted phenol according to claim 1 or 2 wherein the clay mineral is a metal exchange type clay mineral.

5. The process for producing a para-substituted phenol according to claim 1 or 2 wherein the clay mineral is an ion exchange type montmorillonite.

6. The process for producing a para-substituted phenol according to claim 1 or 2 wherein the clay mineral is a metal exchange type montmorillonite.

7. The process for producing a para-substituted phenol according to claim 3 wherein the ion exchange type clay mineral is ion exchabge type montmorillonite expressed by the general formula (4)
(Xa)m1(Xb)m2(Xc₄O₁₀)(Xd)₂ · mH₂O (4)
in the general formula (4), Xa represents at least one ion selected from the group consisting of a cation of the elements of group 1, group 2, group 3, group 4, group 5, group 6, group 7, group 8, group 9, group 10, group 11, group 12, group 13, group 14, or group 15, and ammonium ion; Xb represents at least one atom selected from the group consisting of lithium, magnesium, aluminum, cadmium, lead, zinc, copper, iron, cobalt, nickel, and manganese; Xc represents a silicon atom, a part of which may be replaced by at least one atom selected from the group consisting of aluminum, iron, germanium, titanium, and boron; Xd represents fluorine atom or OH; m1 and m2 are numerical values determined by electrostatic balance, and m1 is a number of 1/5 to 1 and m2 is a number of 1 to 3; and m indicates the amount of water of hydration.

8. The process for producing a para-substituted phenol according to claim 7 wherein Xa in the general formula (4) is at least one cation selected from the group consisting of H⁺, Na⁺, Sn⁺, Ca²⁺, Mg²⁺, Zn²⁺, Ce²⁺, Cu²⁺, Pb²⁺, Ni²⁺, Al³⁺, Fe³⁺, and Zr⁴⁺.

9. A process for producing a para-substituted phenol comprising the step of reacting a cyclohexanone derivative expressed by the general formula (1) with a phenol expressed by the general formula (3) by using an ion exchange type montmorillonite expressed by the general formula (4) as catalyst 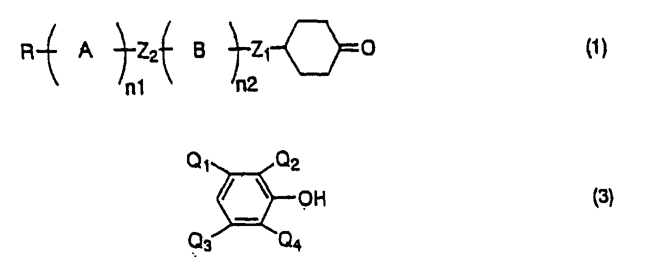 in the general formula (1), A and B each independently represent a carbon six-membered ring in which ring one or more carbon atoms may be replaced by hetero atoms, and one or more hydrogen atoms may be replaced by halogen atoms, alkyl groups, cyano groups, or hydroxyl groups; R represents hydroxyl group, a halogen atom, cyano group, amino group, nitro group, -CH=CF₂, -CH₂CH₂CH=CF₂, or a straight chain or branched alkyl group having 1 to 30 carbon atoms in which alkyl group methylene group may be replaced by oxygen atom, sulfur atom, -COO-, -OCO-, -CH=CH-, or -C≡C-, but there is not a case that oxygen atoms are adjacent, and one or more hydrogen atoms in which group may be replaced by halogen atoms, hydroxyl groups, cyano groups, or nitro groups; Z₁ and Z₂ each independently represent single bond, -CH₂-, -CH₂CH₂-, -CH₂CH₂CH₂-, -CH₂CH₂CH₂CH₂-, -CH(CH₃)CH₂-, -CH₂CH(CH₃)-, -CH₂O-, -OCH₂-, -CH₂CH₂CH₂O-, -OCH₂CH₂CH₂-, -COO-, -OCO-, -CF₂O-, -OCF₂-, -CH=CH, -CF=CF-, -C≡C-, or -S-S-; n1 and n2 are each independently 0 or 1;
in the general formula (3), Q₁ to Q₄ each independently represent hydrogen atom, a halogen atom, an alkyl group, or cyano group;
and
each atom which constitutes these compounds may be replaced by its isotope
(Xa)m1(Xb)m2(Xc₄O₁₀)(Xd)₂ · mH₂O (4)
in the general formula (4), Xa represents at least one ion selected from the group consisting of a cation of the elements of group 1, group 2, group 3, group 4, group 5, group 6, group 7, group 8, group 9, group 10, group 11, group 12, group 13, group 14, or group 15, and ammonium ion; Xb represents at least one atom selected from the group consisting of lithium, magnesium, aluminum, cadmium, lead, zinc, copper, iron, cobalt, nickel, and manganese; Xc represents a silicon atom, a part of which may be replaced by at least one atom selected from the group consisting of aluminum, iron, germanium, titanium, and boron; Xd represents fluorine atom or OH; m1 and m2 are numerical values determined by electrostatic balance, and m1 is a number of 1/5 to 1 and m2 is a number of 1 to 3; and m indicates the amount of water of hydration.

10. The process for producing a para-substituted phenol according to claim 9 wherein Xa in the general formula (4) is at least one cation selected from the group consisting of H⁺, Na⁺, Sn⁺, Ca²⁺, Mg²⁺, Zn²⁺, Ce²⁺, Cu²⁺, Pb²⁺, Ni²⁺, Al³⁺, Fe³⁺, and Zr⁴⁺.

11. A process for producing a para-substituted phenol comprising the step of reacting an aldehyde expressed by the general formula (2) with a phenol expressed by the general formula (3) by using an ion exchange type montmorillonite expressed by the general formula (4) as catalyst. 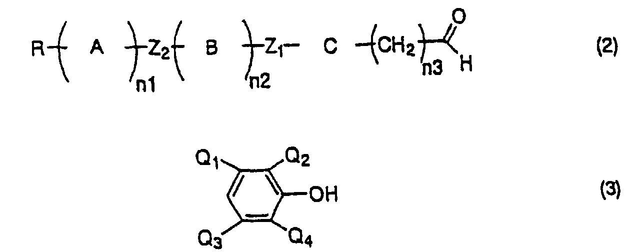 in the general formula (2), A, B, and C each independently represent a carbon six-membered ring in which ring one or more carbon atoms may be replaced by hetero atoms, and one or more hydrogen atoms may be replaced by halogen atoms, alkyl groups, cyano groups, or hydroxyl groups; R represents hydroxyl group, a halogen atom, cyano group, amino group, nitro group, -CH=CF₂, -CH₂CH₂CH=CF₂, or a straight chain or branched alkyl group having 1 to 30 carbon atoms in which alkyl group methylene group may be replaced by oxygen atom, sulfur atom, -COO-, -OCO-, -CH=CH-, or -C≡C-, but there is not a case that oxygen atoms are adjacent, and one or more hydrogen atoms in which group may be replaced by halogen atoms, hydroxyl groups, cyano groups, or nitro groups; Z₁ and Z₂ each independently represent single bond, -CH₂-, -CH₂CH₂-, -CH₂CH₂CH₂-, -CH₂CH₂CH₂CH₂-, -CH(CH₃)CH₂-, -CH₂CH(CH₃)-, -CH₂O-, -OCH₂-, -CH₂CH₂CH₂O-, -OCH₂CH₂CH₂-, -COO-, -OCO-, -CF₂O-, -OCF₂-, -CH=CH-, -CF=CF-, -C≡C-, or -S-S-; n1 and n2 are each independently 0 or 1;
in the general formula (3), Q₁ to Q₄ each independently represent hydrogen atom, a halogen atom, an alkyl group, or cyano group;
and
each atom which constitutes these compounds may be replaced by its isotope
(Xa)m1(Xb)m2(Xc₄O₁₀)(Xd)₂ · mH₂O (4)
in the general formula (4), Xa represents at least one ion selected from the group consisting of a cation of the elements of group 1, group 2, group 3, group 4, group 5, group 6, group 7, group 8, group 9, group 10, group 11, group 12, group 13, group 14, or group 15, and ammonium ion; Xb represents at least one atom selected from the group consisting of lithium, magnesium, aluminum, cadmium, lead, zinc, copper, iron, cobalt, nickel, and manganese; Xc represents a silicon atom, a part of which may be replaced by at least one atom selected from the group consisting of aluminum, iron, germanium, titanium, and boron; Xd represents fluorine atom or OH; m1 and m2 are numerical values determined by electrostatic balance, and m1 is a number of 1/5 to 1 and m2 is a number of 1 to 3; and m indicates the amount of water of hydration.

12. The process for producing a para-substituted phenol according to claim 11 wherein Xa in the general formula (4) is at least one cation selected from the group consisting of H⁺, Na⁺, Sn⁺, Ca²⁺, Mg²⁺, Zn²⁺, Ce²⁺, Cu²⁺, Pb²⁺, Ni²⁺, Al³⁺, Fe³⁺, and Zr⁴⁺.
